# EUROPEAN PATENT SPECIFICATION

(11) **EP 4 188 471 B1**
(45) Date of publication and mention of the grant of the patent: **03.07.2024**
(21) Application number: 21763009.4
(22) Date of filing: 28.07.2021
(51) Int. Cl.: A61L 29/16, A61L 31/16

(54) **MICROSPHERE DRUG COATED MEDICAL DEVICES AND METHODS**
MIT MIKROKUGELN BESCHICHTETE MEDIZINISCHE VORRICHTUNGEN UND VERFAHREN
DISPOSITIFS MÉDICAUX REVÊTUS DE MÉDICAMENT MICROSPHÉRIQUE ET PROCÉDÉS

(30) Priority: 29.07.2020 US 202063058331 P; 19.10.2020 EP 20202626
(43) Date of publication of application: 07.06.2023
(73) Proprietor: BIOTRONIK AG, 8180 Bülach (CH)
(72) Inventor: BETTS, Ronald, E., La Jolla, CA 92037 (US); NAGUI, Ana, Jhelynne, P., La Jolla, CA 92037 (US); NGUYEN, John, Dang, La Jolla, CA 92037 (US); QUACH, David, La Jolla, CA 92037 (US)
(74) Representative: Biotronik Corporate Services SE
(86) International application number: PCT/EP2021/071150
(87) International publication number: WO 2022/023416

(56) References cited:
- US-A1- 2012 015 019
- US-A1- 2018 200 413

## Description

### FIELD OF THE INVENTION

This application relates generally to the field of materials and manufacturing methods involving anhydrous single or multi-solvent, excipient and polymer-free drug coating formulations for production of microsphere coated drug delivery techniques.

### BACKGROUND OF THE INVENTION

Balloon angioplasty is an established method for treatment of various arterial disease conditions. The advent of stents and drug releasing stents however pushed balloon angioplasty into the background. Recently, drug coated angioplasty balloons (DCB) have emerged as a viable alternative to non-drug coated balloons "POBA" (plain old balloon angioplasty). This emergence was initially fueled by clinical data supporting the use of angioplasty balloons coated with the antiproliferative paclitaxel incorporated with an excipient to treat in-stent restenosis. (Chow et al., Interv Cardiol 7(2):169-180 (2015))

A variety of paclitaxel drug forms, excipients, balloons and coating methods have been employed for treatment in human clinical trials with very mixed results. (Picard et al., Archives of Cardiovascular Disease 110:259-272 (2017)) More recently sirolimus has been suggested for DCB use. Both paclitaxel and sirolimus can be effective in preventing restenosis but differ in their biochemical mechanisms. Studies have shown that paclitaxel is potentially more toxic and with a more narrow therapeutic range. (Pires et al., Heart 93:722-727 (2007)) This understanding has resulted in great interest and many investigations evaluating sirolimus use with both vascular stents and drug coated balloons.

It is now generally accepted that sirolimus and sirolimus derivatives ("limus") compounds are the preferred drug for use on coated vascular stents. The industry's desire for development of limus coated balloons has been more challenging, however. It is now understood that for optimum results with these compounds, there is a requirement for drug to remain in the diseased tissue at therapeutic levels for approximately one month or longer. In attempts to accommodate this requirement, a variety of polymers and excipients have been utilized with these drugs as well as a variety of component physical forms, all trying to avoid or to slow drug loss from the balloon during insertion, to allow for drug transfer and retention at the site needed, and to minimize drug washout and/or provide therapeutic amounts of drug vs. time to the tissue.

These requirements have led to complex limus DCB coating compositions and manufacturing methods. (Arpit et al., Trends Biomat Artif Organs 22(2):87-92 (2008) It is also now further realized that DCB coatings containing ingredients in microparticulate and/or microspherical form can aid in drug retention and timed release at treatment sites. These product requirements have resulted in the disclosure of a wide variety of drug forms and compositions.

Table 1 below lists several limus coated balloon designs and their contained required coating additives.

**Table 1**

| Limus coated balloon desisns | | |
|---|---|---|
| **Drug** | **Drug Physical Form** | **Additive** |
| Sirolimus (SIR) | Amorphous | Probucol ¹ |
| SIR | Amorphous and Crystalline | Butylated hydroxyl toluene (BHT) ¹ |
| SIR | Particulate | Cationic excipient ² |
| SIR | Particulate | Cationic block copolymer comprising charged blocks and uncharged blocks ³ |
| SIR | Particulate | Poly(alkyleneimines, axylated quaternary ammonium salts alkylsubstituted chromanols ^{4,5} |
| SIR | Microparticles & Microspheres | Two polymers ^{6,7} |
| SIR | Spherical | Lipoid E80 ⁸ |
| SIR | Microspheres | Cholesterol, biodegradable or bioerodable polymer and phospholipid with acyl chain of 20 to about 34 carbon atoms ^{9,10} |
| Everolimus | Not Reported | Excipient and a plasticizer ¹¹ |
| Zotarolimus | Not Reported | Excipient and a plasticizer ¹¹ |
| Zotarolimus | Not Reported | Synthetic polycationic polymer ¹² |

| | | |
|---|---|---|
| 1. Claver et al., Circ Cardiovasc Interv 9:4e003543 (Apr 2016). 2. U.S. 10,213,529 Surmodics 3. U.S. 10,213,528 Surmodics 4. U.S. 9,949,957 Surmodics 5. U.S. 10,449,180 Surmodics 6. U.S. 10,188,772 Micell Tech 7. U.S. 10,117,972 Micell Tech 8. U.S. 8,778,379 Concept Medical 9. U.S. 9,492,594 MedAlliance 10. U.S. 10,098,987 MedAlliance 11. U.S 2014/0046254 Abbott 12. U.S. 9,782,516 Abbott | | |

In-situ balloon formation of drug microspheres has been previously reported. For example, U.S. 9,295,663 discloses several advantages of in-situ balloon microsphere formation over conventional balloon microsphere coating formulations. However, the disclosed materials and methods, required compositions and necessary processes are very complex. These materials and methods require the balancing of specific chemicals, as well as their chemical and physical properties, together with use of two dissimilar solvents, after which the technique utilizes specific drying requirements in order to produce the desired outcome.

Such teachings of the art require that optimization of traditional drug coating formulations would fail to produce a coating in which microspheres are formed in situ. Furthermore, the relevant art requires that for drug microspheres to form properly, the formulations must utilize water as the first solvent at a ratio of 30% of the second solvent. More importantly, if evaporation of the coating formulation is too fast, microspheres of the drug will fail to form.

US 2012/015019 discloses a method for coating a medical device comprising forming a homogeneous coating solution by dissolving a hydrophobic drug having a solubility in phosphate buffered saline of about 1mg/ml or less than 1 mg/ml in a coating solvent comprising one or more first solvents in which the drug has a solubility of no more than 2000mg/liter and one or more second solvents in which the drug has a solubility of not less than 10,000 mg/liter, wherein the one or more solvents is/are more volatile than the one or more first solvents by at least a factor of 2; disposing the coating solution over a surface of the medical device; and removing the one or more second solvents at a temperature and pressure selected such that the drug precipitates from the remaining coating solution as particles of a volume weighted average diameter of 30 microns or less; and removing the remaining coating solvent. The medical device is an expandable medical device e.g. a balloon catheter.

It can be realized that the utilization of polymers, extraneous additives, and excipients to result in an optimum physical and chemical drug product configuration that satisfies clinical requirements is challenging. Various product additives can greatly increase manufacturing complexities and costs and can also negatively affect required packaging and stability (shelf life) of the product. Also, with added product components there is an increased risk of patient vascular and/or systemic toxicities, all affecting patient outcomes and total treatment cost. There is a clear need in the art for improved limus DCB compositions of matter and manufacturing processes.

### SUMMARY OF THE INVENTION

The invention is defined by the claims.

Therefore, one aspect of the present invention is directed to a medical device having a coating layer comprising or consisting of a plurality of drug microspheres as described and defined herein.

Related to the previous aspect the present invention is directed to a medical device, preferably a balloon catheter having a balloon with a coating layer comprising or consisting of a plurality of drug microspheres as described and defined herein. The medical device above is directed to a balloon catheter, having a balloon with a primer coating layer, comprising a water soluble material and a second layer comprising or consisting of a plurality of drug microspheres as described and defined herein. A primer layer as used herein is supposed to be applied on the substrate's surface. For example, the primer layer of a balloon catheter is applied on the balloon surface.

Also, the present invention provides a method of manufacturing drug microspheres comprising at least one drug, comprising the steps of (1) dissolving the at least one drug in at least one solvent to form a hydrophobic mixture, (2) depositing the dissolved hydrophobic mixture onto a substrate and (3) evaporating the deposited, dissolved hydrophobic mixture from the substrate to yield the drug microspheres. The method as described above includes an intermediate step (1b) applied previous to step (1), where step (1b) comprises applying a water soluble material to the substrate and drying the water soluble material. The application of step (1b) forms a primer layer of water soluble material on the substrate. Subsequent step (2) is hence conducted that the dissolved hydrophobic mixture is deposited onto the substrate having a primer layer.

### BRIEF DESCRIPTION OF THE DRAWINGS

The disclosed subject matter contained herein is best described in conjunction with the accompanying drawings, in which:
Figure 1 shows SEM images for each coated balloon. (A) 3.1 mg/mL CRC-015 in anhydrous methanol coated on balloon; (B) 6.3 mg/mL CRC-015 in anhydrous methanol coated on balloon; (C) 12.5 mg/mL CRC-015 in anhydrous methanol coated on balloon; and (D) 25.0 mg/mL CRC-015 in anhydrous methanol coated on balloon.
Figure 2 shows paclitaxel spheres coated on a balloon. (A) image of the balloon as coated with paclitaxel formulation in methanol at 25 mg/mL; (B) SEM image of the drug microspheres.
Figure 3 shows CRC-015, paclitaxel, and gemcitabine spheres coated on a balloon. (A) image of the balloon as coated with CRC-015, paclitaxel, and gemcitabine dissolved together in methanol; (B) SEM image of the drug formulation microspheres.
Figure 4 shows microspheres as produced with CRC-015 in methanol (37.5 mg/mL) and 5% water.
Figure 5 shows microspheres as produced with rapamycin in methanol (25 mg/mL) and 7% water.
Figure 6 shows microspheres as produced with paclitaxel in methanol (10 mg/mL) and 4% water.
Figure 7 shows microspheres as produced with everolimus in ethanol (45 mg/mL) and 1% water.
Figure 8 shows microspheres produced with formulation comprising everolimus and alisertib in 34.2% methanol (v/v) and 56.8% acetone (v/v).
Figure 9 shows microspheres produced with a formulation of CRC-015 in methanol coated on top of HSA.
Figure 10 shows microspheres produced with a formulation of CRC-015 in methanol coated on top of PVA.
Figure 11 shows in vivo data supporting arterial vessel drug retention comparison of CRC-015 DCB versus sirolimus DCB versus MagicTouch sirolimus DCB.

### DETAILED DESCRIPTION OF THE INVENTION

### Definitions

As used herein, the term "macrocyclic triene immunosuppressive compound" includes rapamycin (sirolimus), everolimus, zotarolimus, biolimus, novolimus, myolimus, temsirolimus and the rapamycin derivatives described in this disclosure.

As used herein, the term "microsphere" or "microspherical" includes small, largely spherical particles with diameters in the nanometer to micrometer range. The term "microsphere" or "microspherical" includes both a micellar structure being hollow as well as a ball structure being filled with material down to the core without having any particular hollow space inside. The term is directly referred to the term "particle" which can also have the meaning of being partly hollow or completely filled.

The present invention provides for materials and methods for the novel production of drug microspheres for use in treating certain diseases or conditions.

One aspect of the present invention provides a method of manufacturing drug microspheres comprising at least one drug, comprising the steps of (1) dissolving the at least one drug in at least one solvent to form a hydrophobic mixture, (2) depositing the dissolved hydrophobic mixture onto a substrate and (3) evaporating the deposited, dissolved hydrophobic mixture from the substrate to yield the drug microspheres. The method as described above includes an intermediate step (1b) applied previous to step (2), where step (1b) comprises applying a water soluble material to the substrate and drying the water soluble material. The water soluble material for this step can be any material as defined herein as water soluble material. It is preferred that the substrate is a medical device such as a balloon catheter or a stent, but could also be a pacemaker, an implantable defibrillator or implantable neuro stimulating device. Also, the method as described herein may include a step (1a) being applied previously to step (1) or step (1b) if present. The step (1a) provides for cleaning and wetting the substrate, preferably the balloon of a balloon catheter, with an organic solvent as described herein. Preferably, step (1a) is conducted with acetone, pentane, hexane, ethanol, diethyl ether or ethyl acetate. Conducting step (1a) has the advantage that either the water soluble material or the drug microsphere show an improved adhesion to the substrate. Also, prior to depositing the dissolved hydrophobic mixture onto the substrate, the substrate may be cleansed by a plasma treatment. Preferably, the plasma treatment step is conducted after cleaning and before applying step (1b). Plasma treatment may support the dissolution of the water soluble material and thereby faster transfer of the at least one drug.

Certain advantages of the preferred embodiments of the present invention include, but are not limited to, increasing drug yield (even when using lipophilic drug compounds) on the milligram scale, reducing the complexities associated with microsphere formation (including reduction of staff training and expensive equipment requirements), dramatically reducing processing time (resulting in diminishing likelihood of drug breakdown and lowering procedure expenses) and removing expensive materials (such as volatile compounds and costly synthetic polymers) from the productions steps.

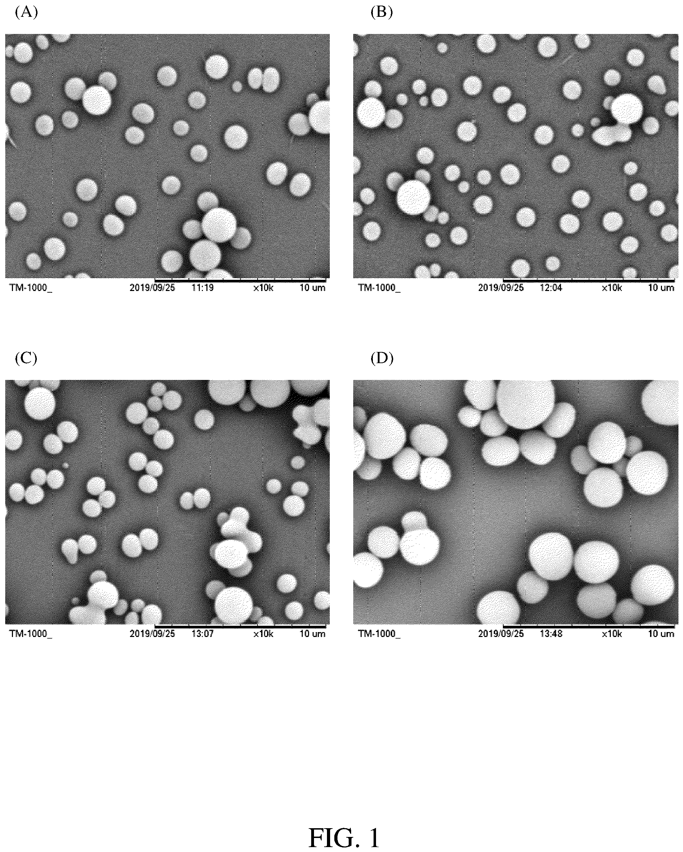

The present invention relates to methods and processing steps that provide for the formation of drug microspheres after solubilizing at least one drug or drug composition (or multiple drug compositions) in at least one solvent or various solvents. This solubilizing or dissolving step is followed by dispensing the resulting mixture onto a substrate. FIG. 1 illustrates one embodiment of how the mixture is dispensed onto a particular substrate. Preferably, a syringe is utilized to dispense the dissolved drug mixture onto a substrate. Alternatively, a pipette or a needle or cannula may be used in a similar fashion.

As stated above various solvents can be used for the present application. Preferred solvents are organic solvents which have been dried prior to use. Preferably only one solvent is used for the solubilizing or dissolving step, but also various solvents, hence solvent mixtures can be used. The solvents shall be dry. A dry solvent is to be understood that measures have been applied to remove water from it. The extent of the removal can usually range from a simple distillation of the solvent to treating and storing the solvent after or during distillation with a drying agent such as sodium, potassium, NaAlH₄ and the like. Preferably, the solvent contains less than 7% water, more preferably less than 3% water, even more preferably less than 1% water and most preferably less than 0.01% water. In that it is preferred, if water is present in the solvents in amounts up to a maximum of about 7%. In that preferred embodiment an unmet need for a solution in the art is satisfied which utilizes moisture-free formulations containing a single or multi-solvent that will rapidly form drug microspheres and, if water is present in amounts up to about 7%, the microsphere formation still rapidly proceeds at ambient temperature.

It is even more preferred if the solvents do have a water content below 1%. Suitable solvent are organic solvents such as alcohols, in particular, C1 to C3 aliphatic alcohols, acetone, ethers, in particular diethyl ether, chloroform, dichloromethane, acetonitrile, hexane and pentane. Particularly preferred are C1 to C3 alcohols, for instance methanol or ethanol.

After depositing the solvent-drug mixture onto the substrate, evaporation of the mixture results in formation of drug microspheres on the substrate surface. Optimization of production conditions may result in specific improvements to the final product, for example, control of sphere size or automation of the processing steps. It is preferred that the step of evaporating the of the at least one solvent occurs without means for accelerating evaporation, for example by slight wind or stream of air. If evaporation occurs slowly a narrow particle size distribution is observed. In case strong means for accelerating evaporation is applied like a stronger gas stream, heating the solvent close or above the boiling temperature of the at least one solvent formation of microspherical hydrophobic material is not observed. In terms of a gas stream it is preferred if gas is moving over the surface of the solution slower than 0.2 m/s.

The concentration of the hydrophobic drug composition in the at least one solvent is supposed to be in a range of 0.1 to 200 mg/ml. In a preferred embodiment the concentration is in the range of 10 to 100 mg/ml. In the aforementioned range a narrow particle size distribution is observed. Also, in case of a higher drug concentration as defined above better adhesion of the microspherical hydrophobic material on the substrate could be observed. Without being bound to the following theory it is believed that with increasing concentration the microspherical particles tend to become bigger in diameter and with the increased diameter the surface area contributing to the adhesion between particle and surface is also increased.

Preferably, the at least one drug as used in the present invention is a macrocyclic triene immunosuppressive compound selected from the group consisting of rapamycin (sirolimus), everolimus, zotarolimus, biolimus, novolimus, myolimus, temsirolimus and rapamycin derivatives, including any pharmaceutically acceptable salts and hydrates, as well as any stereoisomers, mixtures of stereoisomers and racemates. More preferably, the macrocyclic triene immunosuppressive compound, including any pharmaceutically acceptable salts and hydrates, as well as any stereoisomers, mixtures of stereoisomers and racemates, has the following structure: 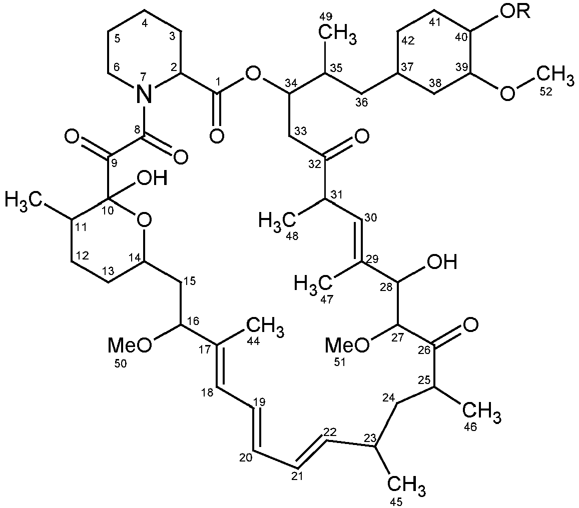 where R is C(O)-(CH₂)ₙ-X, n is 0, 1 or 2, X is a cyclic hydrocarbon having 3-8 carbons and optionally contains one or more unsaturated bonds. In a most preferred embodiment, C(O)-(CH₂)ₙ-X has one of the following structures: 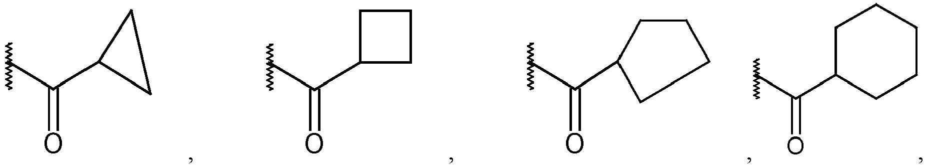 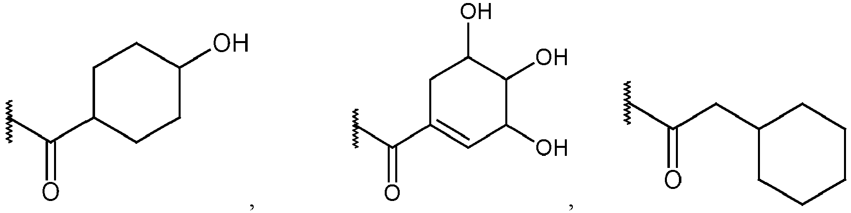 and

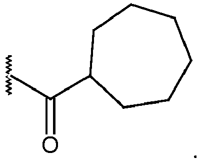

In one embodiment the drug microspheres consist of the macrocyclic triene immunosuppressive compound, including any pharmaceutically acceptable salts and hydrates, as well as any stereoisomers, mixtures of stereoisomers and racemates, as defined above. In another embodiment the drug microspheres comprise the macrocyclic triene immunosuppressive compound, including any pharmaceutically acceptable salts and hydrates, as well as any stereoisomers, mixtures of stereoisomers and racemates, as defined above. One particularly preferred embodiment includes drug microspheres being formed from the macrocyclic triene immunosuppressive compound, including any pharmaceutically acceptable salts and hydrates, as well as any stereoisomers, mixtures of stereoisomers and racemates, as defined above and alisertib. The ratio between the two compounds varies from 5: 95 by weight to 95:5 by weight. In a preferred embodiment the drug microspheres have a content of alisertib being less than 50% by weight.

A further preferred embodiment is directed to drug microspheres including or consisting of rapamycin or derivative thereof as defined herein and Paclitaxel. In a specific embodiment the drug microspheres consist of paclitaxel and the macrocyclic triene immunosuppressive compound, including any pharmaceutically acceptable salts and hydrates, as well as any stereoisomers, mixtures of stereoisomers and racemates, having the following structure: 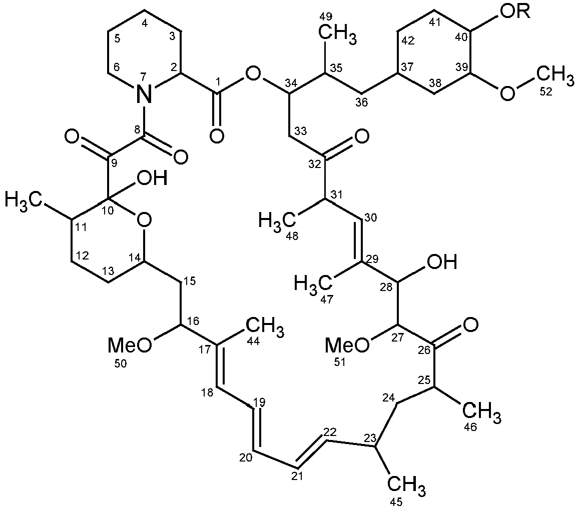 where R is C(O)-(CH₂)ₙ-X, n is 0, 1 or 2, X is a cyclic hydrocarbon having 3-8 carbons and optionally contains one or more unsaturated bonds. In a most preferred embodiment, C(O)-(CH₂)ₙ-X has one of the following structures: 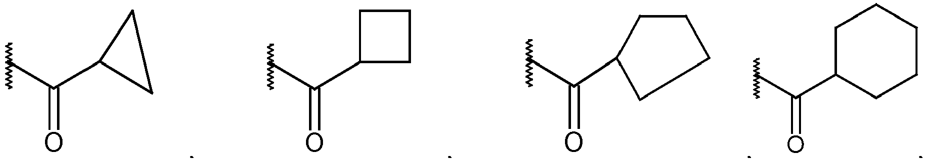 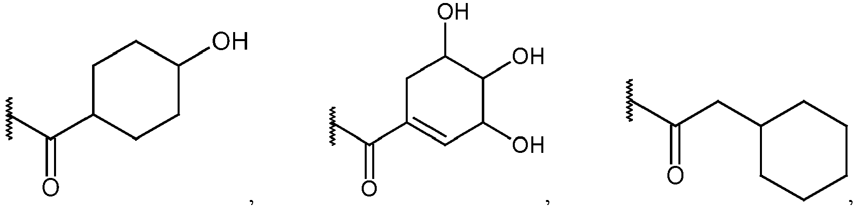 and

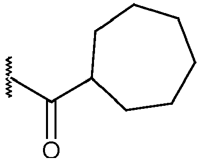

One further aspect of the present invention is directed to a medical device, preferably a balloon catheter, having a coating layer, comprising or consisting of a plurality of drug microspheres as described and defined herein. One preferred embodiment of the medical device above is directed to a balloon catheter, having a balloon with a primer coating layer, comprising a water soluble material and a second layer comprising or consisting of a plurality of drug microspheres as described and defined herein. A primer layer as used herein is supposed to be applied on the substrate's surface. For example, the primer layer of a balloon catheter is applied on the balloon surface.

It is within the meaning of the present invention to provide medical devices having microspheres as described herein as an outside layer in order to facilitate drug transfer from the medical device to patient's tissue. A primer layer is introduced between the surface of the medical device and the layer of microspheres.

A primer coating layer is comprised or consists of a water soluble material and forms the first coating layer on the surface of the balloon catheter. This primer coating layer is preferably applied via dip coating, wherein the balloon catheter is placed into a solution comprising the water soluble material, which is applied to the surface of the balloon catheter. Other suitable methods such as spraying or application of a solution by a thread, a needle, a cannula, a sponge or a piece of cloth can be used for the coating procedure. Following this application of the primer coating layer, the balloon catheter is removed from the solution or the application device and allowed to dry, for example at ambient room temperature for a time less than 24 hours

The water soluble material that is meant to comprise the bulk of the primer coating layer is selected from water soluble human serum proteins preferably having an approximate molecular weight of between 50 to 200 kD. In one embodiment the water soluble material is a protein having an approximate molecular weight of between 65-70 kD, preferably a globular serum protein having an approximate molecular weight of between 65-70 kD. In a further embodiment the water soluble material is selected from blood proteins such as globulins and/or fibrinogens having molecular weights up to approximately 160 kD. More preferably, the water soluble material is human fibrinogen or immunoglobulin. Most preferably, the water soluble material is a human serum protein having at least 90% identity to the following sequence:












 (SEQ ID NO: 1)

In a most preferred embodiment of the invention the water soluble material is human serum albumin. The use of human serum albumin is preferred in that the compound has human origin and thereby does not contribute to an unnecessary drug or compound load for a patient being treated with a balloon catheter as described herein. Thereby, the use of human serum albumin reduces unnecessary contamination of patients. Human serum albumin when used and applied as primer layer may include minor amounts of stabilizing compounds in the range of impurities. In embodiments where the primer layer is supposed to consist of human serum albumin or any other suitable protein it is within the meaning of the present invention and the embodiments presented herein that those minor amounts are supposed to be covered by a wording "consisting of'.

As described above the drug microspheres are small, largely spherical particles with diameters in the nanometer to micrometer range. In one embodiment, the drug microspheres are in the micrometer range and have an average diameter of 1 to 20 µm. In preferred embodiment, the drug microspheres have an average diameter of 3 to 10 µm. In such a range a synergistic effect could be observed when used on a balloon catheter in that the microsphere show strong adhesion to the substrate while at the same time the drug elution to the tissue was very effective. Without being bound to this theory it is believed that diameters being clearly out of the nanoscope have a comparatively high surface area contributing to the adhesion between particle and surface while still having an overall great surface area which is necessary for an effective drug transfer.

Also as described above the load of foreign compounds to the patient is kept at a minimum by preferably using no other layers than the drug microsphere layer or additionally a layer of human serum albumin. Especially in these embodiments in order to provide an efficient implantation procedure with an effective drug elution/transfer it can be afforded to apply a higher drug dose than with other layer combinations where usually binders, excipients or surfactants are applied in addition to the API. For example, usual drug loads on balloon catheters are kept between 0.3 and 1.2 mg/mm². As the contamination with the embodiments as described herein is low, higher drug load between 1.5 to 3.0 mg/mm² can be applied.

In the embodiment, where the medical device is a balloon catheter it could be observed that improved adhesion of the particles occurred when the balloon is made of a polyamide material, in particular when the drug microspheres comprise at least one compound which are described herein as CRC-15 species (see table 1 below). Balloon material showing good adhesion comprise polyamides like nylon 6, nylon 66, nylon 11, nylon 12, block copolymers include segmented polyamide-polyether-polyesters sold under the Pebax^{®} trademark, or nylon elastomers, which are block copolymers having nylon 6, nylon 66, nylon 11, nylon 12. The combination of a polyamide based balloon material and drug microspheres comprising or consisting of a CRC-15 species is advantageous as the microsphere particles show good adhesion whereas the polyamide materials have an improved flexibility in comparison to e.g. polycarbonates. In this, a balloon catheter having a balloon made from a polyamide based material and having a microsphere coating as described herein, in particular a microsphere drug coating comprising or consisting of at least one CRC-15 species as described herein have an improved profile for being used for small and curvy vessel such as coronary vessel, because the flexibility of material allows the passage to those vessel while the good adhesion minimizes loss of material to these difficult to reach locations. Also, in the embodiment of absence of a primer layer the balloon catheter exhibits a very small diameter supporting the passage to very small locations.
Therefore, in one particularly embodiment, the present invention is directed to a balloon catheter having a balloon being made of a polyamide material as defined herein having a coating layer applied to the balloon surface comprising or consisting of rapamycin (sirolimus), everolimus, zotarolimus, biolimus, novolimus, myolimus, temsirolimus and/or rapamycin derivatives, including any pharmaceutically acceptable salts and hydrates, as well as any stereoisomers, mixtures of stereoisomers and racemates as well as a macrocyclic triene immunosuppressive compound, including any pharmaceutically acceptable salts and hydrates, as well as any stereoisomers, mixtures of stereoisomers and racemates, having the following structure: 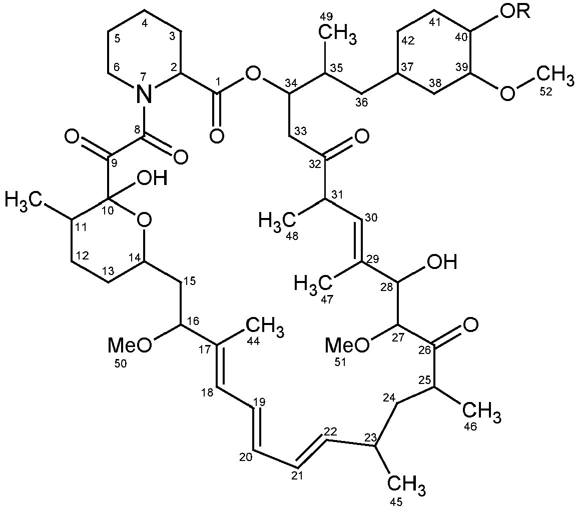 where R is C(O)-(CH₂)ₙ-X, n is 0, 1 or 2, X is a cyclic hydrocarbon having 3-8 carbons and optionally contains one or more unsaturated bonds. In a most preferred embodiment, C(O)-(CH₂)ₙ-X has one of the following structures:

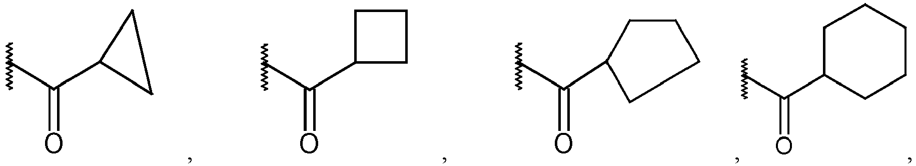

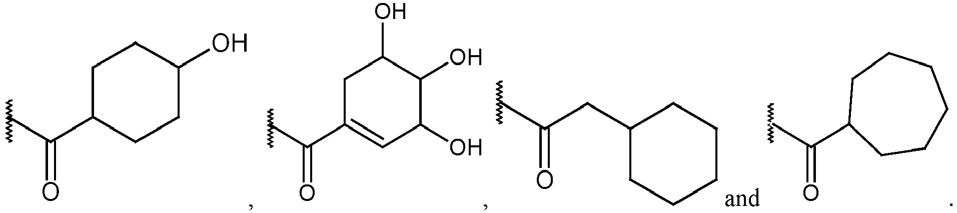

Most preferably in this embodiment the drug comprises or consists of macrocyclic triene immunosuppressive compound, including any pharmaceutically acceptable salts and hydrates, as well as any stereoisomers, mixtures of stereoisomers and racemates, as defined directly above. In one embodiment the present invention is directed to a balloon catheter having a coating layer applied to the balloon surface, the coating layer consisting of drug microspheres, wherein the drug microspheres consist of a macrocyclic triene immunosuppressive compound, including any pharmaceutically acceptable salts and hydrates, as well as any stereoisomers, mixtures of stereoisomers and racemates, having the following structure: 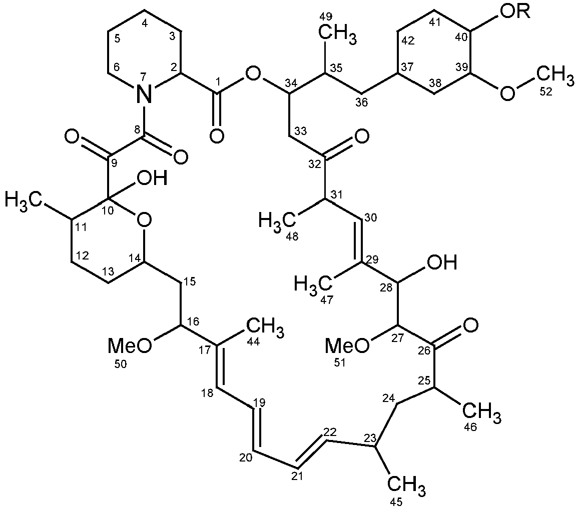 where R is C(O)-(CH₂)ₙ-X, n is 0, 1 or 2, X is a cyclic hydrocarbon having 3-8 carbons and optionally contains one or more unsaturated bonds. In a most preferred embodiment, C(O)-(CH₂)ₙ-X has one of the following structures: 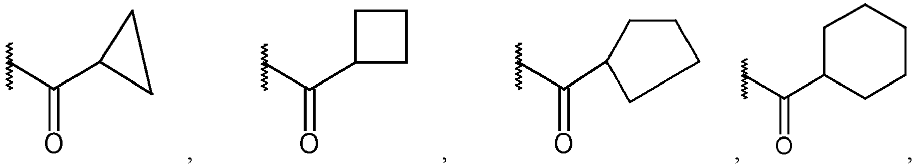 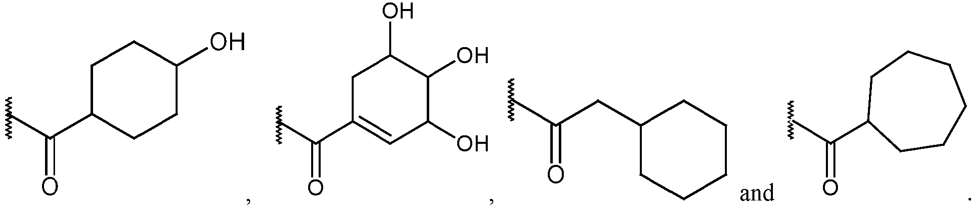 This particular embodiment of a balloon catheter has only one single layer on the balloon surface consisting of microspheres as defined directly above. This embodiment may further have the limitations in terms of balloon materials, particle size and drug load on the balloon as defined herein.
A preferred embodiment is directed to a balloon catheter having a primer layer and a coating layer consisting of drug microspheres. The coating layer is applied on top of the primer layer. The primer layer is applied on the surface of the balloon of the balloon catheter. The primer layer consists of a water soluble material, preferably a protein preferably having a molecular weight of between 50 to 200 kD and most preferably human serum albumin. The drug microspheres consist of a macrocyclic triene immunosuppressive compound, including any pharmaceutically acceptable salts and hydrates, as well as any stereoisomers, mixtures of stereoisomers and racemates, having the following structure: 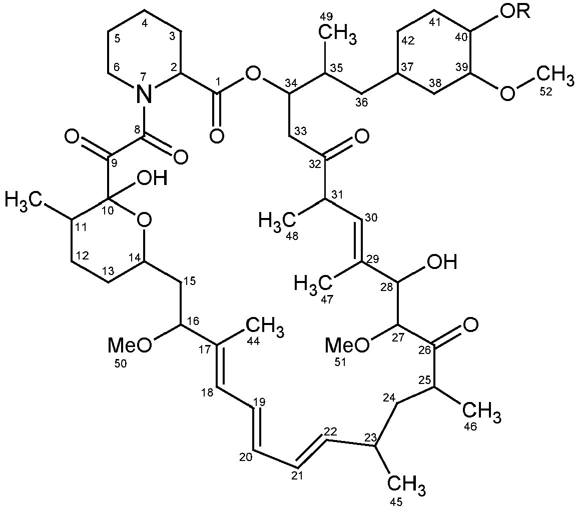 where R is C(O)-(CH₂)ₙ-X, n is 0, 1 or 2, X is a cyclic hydrocarbon having 3-8 carbons and optionally contains one or more unsaturated bonds. In a most preferred embodiment, C(O)-(CH₂)ₙ-X has one of the following structures: 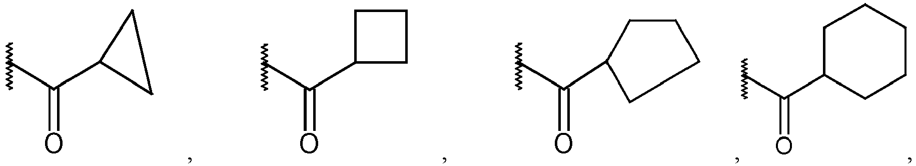 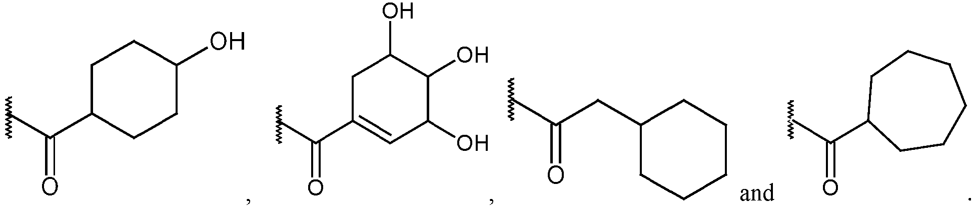 This particular embodiment is supposed to only have the two layers as defined directly above. No further layers are supposed to applied to the balloon. This embodiment may further have the limitations in terms of balloon materials, particle size and drug load on the balloon as defined herein.

### Examples

Examples I-XIII are not according to the invention.

### Example Drug Formulations:

The macrocyclic triene immunosuppressive compound of the present invention has more than one embodiment and may be described as comprising at least one of the following species from Table 1:

**Table 1**

| Description of CRC-015 species | | |
|---|---|---|
| Main structure | R is C(O)-(CH2)n-X having one of the following structures | Species |
| 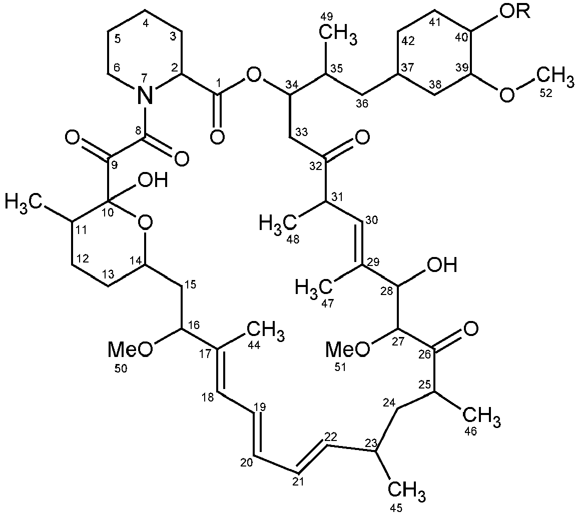 | 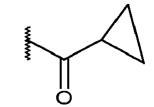 | CRC-015a |
| | 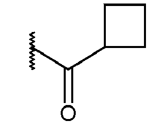 | CRC-015b |
| | 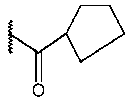 | CRC-015c |
| | 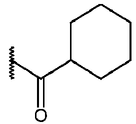 | CRC-015d |
| | 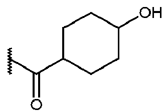 | CRC-015e |
| | 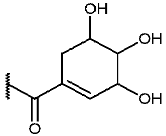 | CRC-015f |
| | 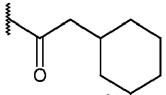 | CRC-015g |
| | 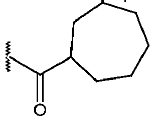 | CRC-015h |

CRC-015, as referred to herein, is a term meant to encompass a genus and used to refer to each of the following species from Table 1: CRC-015a, CRC-015b, CRC-015c, CRC-015d, CRC-015e, CRC-015f, CRC-015g, and CRC-015h.

### I. CRC-015 sphere coated balloons

Anhydrous CRC-015 formulations at 25.0, 12.5, 6.3 and 3.1 mg/mL were investigated for the ability to produce spheres by methods of the invention as well as the role of drug concentration versus sphere size.

Materials: Anhydrous Methanol; Sigma 900641-4x2ML sealed glass ampoules, Lot SHBH9540 ( 0.001% water, Karl Fischer CERT). 4 mL glass vials; heat treated at 140°C overnight before capping with plastic PTFE lined caps. Stainless steel spatula and 1 mL glass pipettes; heat treated as for glass vials. CRC-015; 0.47% moisture (Karl Fischer). 5.0 x 40 mm Passeo 35 balloon catheter (Biotronik Inc.).

Formulation Methods: Anhydrous formulations of CRC-015 were prepared by adding the required drug amount to tared vials using the stainless steel spatula. Required anhydrous methanol volume was added using glass measuring pipette and the vial was immediately capped. Drug was dissolved by brief vortexing of the vial.

Balloon Coating Materials: Coating Equipment; Adept Engineering Limited, PIN 768-01, Syringe Pump; Sono-tek Syringe Pump Ti PIN 12-05-00144, Dispensing Cannula; stainless steel 55 mm L x 1.5 mm OD, 0.9 mm ID x 0.7 mm D side hole.

Balloon Coating Methods: Each anhydrous formulation of CRC-015 was coated onto respective inflated 5.0 x 40 mm Passeo 35 balloon. The dispensing cannula comes in direct contact with the balloon at either the proximal or distal end and surfs along the balloon from end to end while by dispensing 50 µL of formulation solution horizontally across the inflated balloon as the balloon spins on its axis. For these examples the following process parameters were utilized: Solution Flow Rate = 0.2 mL/min, Dispense Time = 15 seconds resulting in a wet balloon surface, Spin Rate = 120/min, Spin Time = 240 seconds resulting in solvent evaporation and formation of drug microspheres. The sphere coated balloon was held at ambient temperature before imaging analysis. The coated balloons were imaged using Hitachi TM-1000 Tabletop scanning electron microscope (SEM). The drug coated balloon was sectioned into 3 equal segments: distal, middle, and proximal. Each section was placed on specimen stage then sputter coated with gold. The specimen stage was then placed inside the sample chamber of the SEM for analysis. The sizes of CRC-015 spheres present in the SEM of distal, middle, and proximal sections of the balloon were measured. Representative SEM images for each coated balloon are shown in Fig. 1. The CRC-015 sphere size measurements are detailed in Table 2. No apparent change in the size of CRC-015 spheres was observed when the drug formulation concentration was increased from 3.1 mg/mL to 6.3 mg/mL to 12.5 mg/mL. However, a statistically significant (P < 0.0001 at 95% confidence interval) difference in the size of CRC-015 spheres was observed when the concentration of CRC-015 was increased to 25 mg/mL.

**Table 2**

| CRC-015 sphere size measurements | | | | | | | | | | | |
|---|---|---|---|---|---|---|---|---|---|---|---|
| **CRC-015 Sphere Size Measurements (µM)** | | | | | | | | | | | |
| **Balloon Section** | **3.1 mg/mL CRC-015 Coated on Balloon** | | | **6.3 mg/mL CRC-015 Coated on Balloon** | | | **12.5 mg/mL CRC-015 Coated on Balloon** | | | **25 mg/mL CRC-015 Coated on Balloon** | |
| | **Average (n=10)** | **STD. Dev.** | | **Average (n=10)** | **STD. Dev.** | | **Average (n=10)** | **STD. Dev.** | | **Average (n=10)** | **STD. Dev.** |
| Distal | 1.21 | 0.05 | | 1.18 | 0.02 | | 1.16 | 0.07 | | 2.05 | 0.15 |
| Middle | 1.24 | 0.06 | | 1.14 | 0.04 | | 1.23 | 0.06 | | 2.12 | 0.22 |
| Proximal | 1.25 | 0.05 | | 1.24 | 0.05 | | 1.13 | 0.10 | | 2.21 | 0.17 |
| **Average (n=30)** | **1.23** | | | **1.19** | | | **1.17** | | | **2.13** | |
| **STD. Deviation** | **0.05** | | | **0.06** | | | **0.08** | | | **0.19** | |

### II. Paclitaxel sphere coated balloons

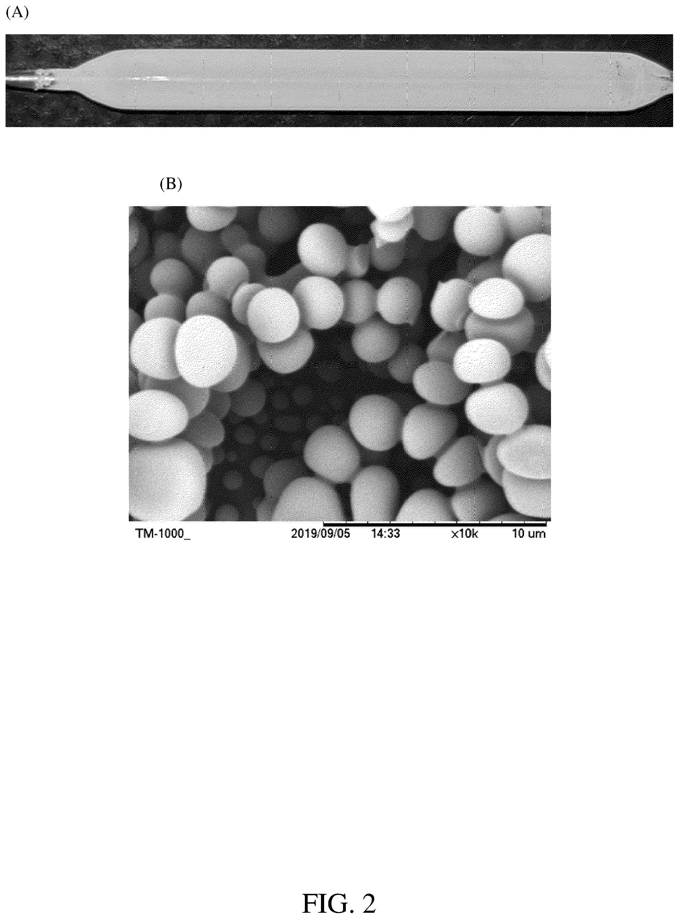

Paclitaxel (LC Laboratories P-3600, Lot ASM-121) was formulated in methanol (EMD MXO488P-1, Lot 59129) 25 mg/mL and the solution was used to coat a balloon in methods similar to Example I. The resulting balloon was imaged (Fig. 2(A)) and resulting SEM analysis showing produced drug microspheres is shown in Fig. 2(B).

### III. Sirolimus sphere coated balloons

Sirolimus (rapamycin) (LC Laboratories R-5000, Lot ASW-134) was formulated as described in Example I and this formulation was utilized to produce drug spheres on the balloon surface.

### IV. Everolimus sphere coated balloons

Everolimus (LC Laboratories E4040, Lot BDE-115) was formulated as described in Example I and this formulation was utilized to produce drug spheres on the balloon surface.

### V. Biolimus sphere coated balloons

Biolimus was formulated as described in Example I and this formulation was utilized to produce drug spheres on the balloon surface.

### VI. Zotarolimus sphere coated balloons

Zotarolimus (Molcan, Lot 10081) was formulated as described in Example I and this formulation was utilized to produce drug spheres on the balloon surface.

### VII. CRC-015 sphere coated balloons

CRC-015 was formulated as described in Example I except 100% ethanol (Sigma 459844, Lot SHBJ8382) was used as the solvent. This formulation was utilized to produce drug spheres on the balloon surface.

### VIII. CRC-015, Paclitaxel, and Gemcitabine sphere coated balloons

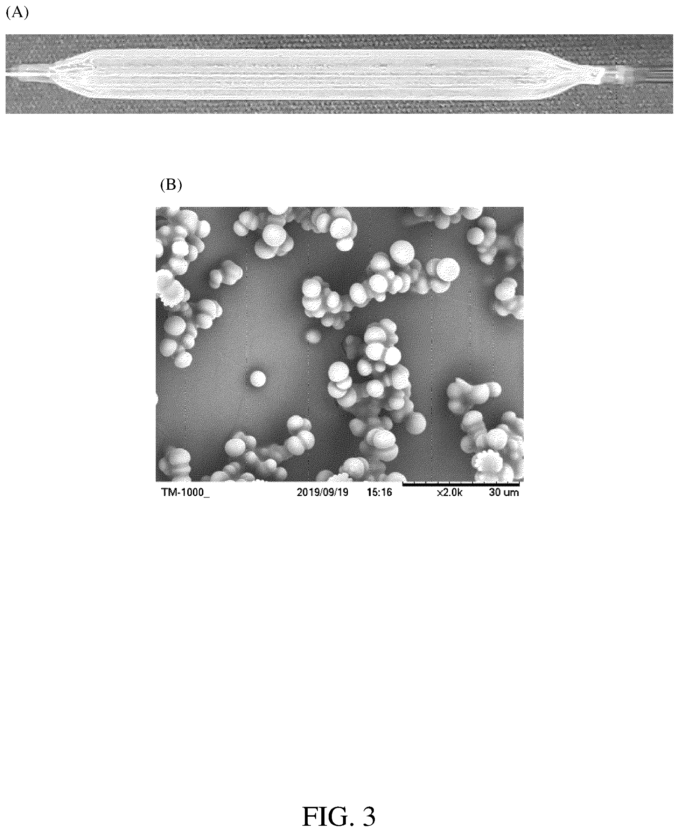

CRC-015, Paclitaxel, and Gemcitabine Free Base (LC Laboratories G4199, Lot GMB-103) were dissolved together in methanol at the following concentrations respectively: 20 mg/ml, 17 mg/ml, 5.2 mg/mL. This solution was used to coat a balloon by the methods of Example I. The resulting balloon was imaged (Fig. 3(A)) and the resulting drug spheres are shown in Fig. 3(B).

The following formulations are examples of coatings containing water.

### IX. CRC-015

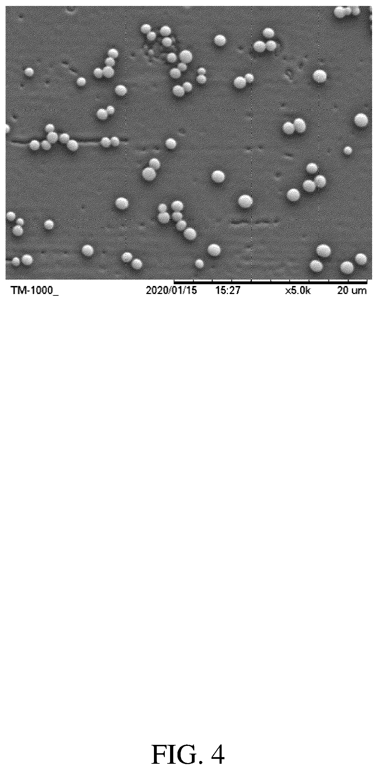

CRC-015 was dissolved in methanol to a concentration of 37.5 mg/mL. HPLC grade water was added to prepare a coating solution containing water at a concentration of approximately 5 % by volume. This coating solution was pipetted onto the surface of a 5 x 120 mm balloon catheter and allowed to dry 30 seconds at ambient conditions. The balloon surface was examined by SEM with resulting SEM image showing drug spheres produced (Fig. 4).

### X. Rapamycin

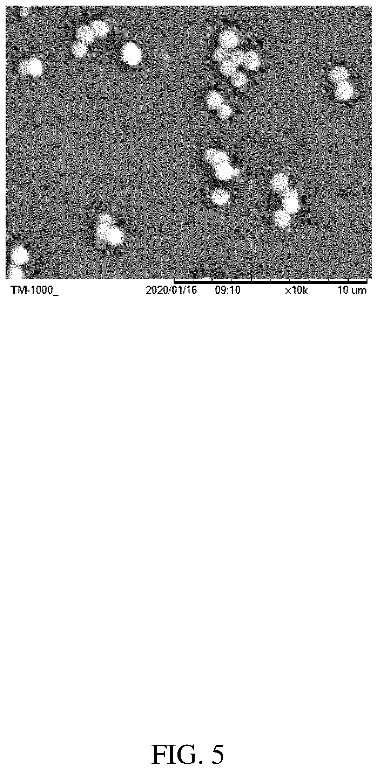

Rapamycin was dissolved in methanol to a concentration of 25 mg/mL. HPLC grade water was added to prepare a coating solution containing water at a concentration of approximately 7 % by volume. Balloon coating and examination was conducted as for Example I. The resulting SEM image showing drug spheres produced is shown (Fig. 5).

### XI. Paclitaxel

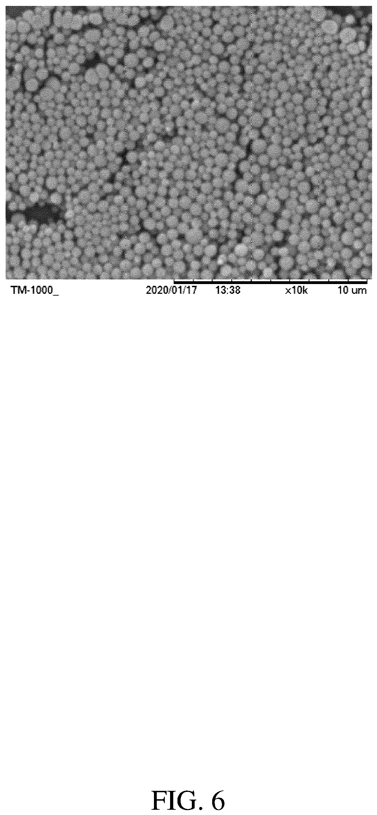

Paclitaxel was dissolved in methanol to a concentration of 10 mg/mL. HPLC grade water was added to prepare a coating solution containing water at a concentration of approximately 4 % by volume. Balloon coating and examination was conducted as for Example I. The resulting SEM image showing drug spheres produced is shown (Fig. 6).

### XII. Everolimus

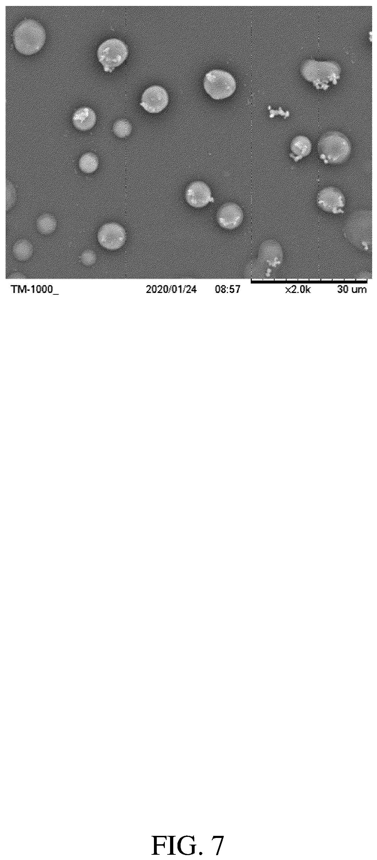

Everolimus was dissolved in 100% ethanol to a concentration of 45 mg/mL. HPLC grade water was added to prepare a coating solution containing water at a concentration of approximately 1 % by volume. Balloon coating was conducted as for Example 1 except solvent drying was 45 seconds followed by SEM analysis. The resulting SEM image showing drug spheres produced is shown (Fig. 7).

These examples of microsphere formulations demonstrate that, although drug microspheres are quickly produced using anhydrous solvent systems, the presence of varying small amounts of water do not inhibit the microsphere formation process.

The following example demonstrates a coating formulation containing multi-solvent and multi-compound mixture for microsphere production.

### XIII. Multi-solvent, multi-compound mixture

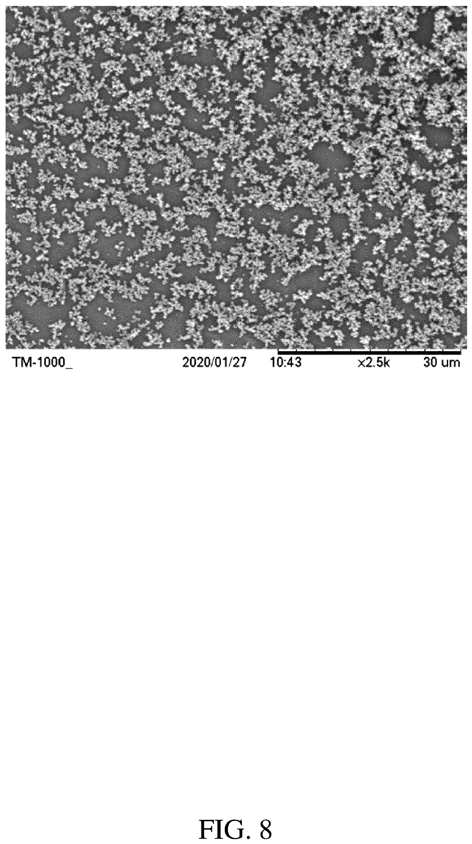

Everolimus and Alisertib (MLN 8237) Adooq Bioscience 1028486-01-2, Lot LI0004B007 were dissolved together in a solution of 34.2% methanol and 65.8% acetone, v/v (EMD) to a concentration of 4.5 mg/mL and 2.5 mg/mL, respectively. The solution was pipetted onto the surface of an inflated balloon and allowed to dry for 30 seconds before SEM analysis. SEM resulting microspheres are shown in Fig. 8.

The following examples show drug microspheres formed utilizing an under layer.

### XIV. Drug microsphere formation with under layer present

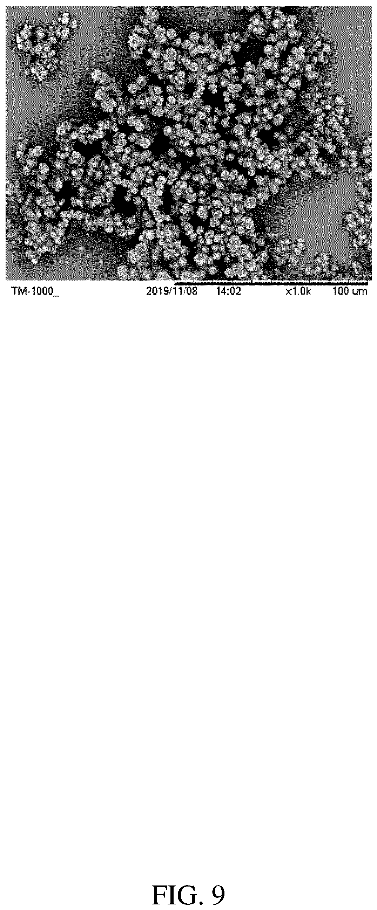

An inflated 5.0 x 40 mm Passeo 35 balloon was cleaned by wiping the surface of the balloon with kimwipes wetted with acetone. The balloon was optionally pre-treated with plasma (w/ argon gas). The dried balloon was dip coated with human serum albumin (HSA) by inserting the balloon, distal end first, into a tube containing a solution of 5% HSA (Albumedix PIN 200-010, Lot 2107, diluted to 5% with HPLC water). The dip coated balloon was removed from the tube and then flipped so the distal end of the balloon is facing up. Excess HSA flowing down to the proximal end of the balloon was removed with kimwipes. The HSA coated balloon was dried at ambient conditions for 14 - 38 hours. CRC-015 was formulated and coated onto the HSA coated balloon as described in Example I. The balloon surface was examined by SEM with resulting SEM image showing drug spheres produced shown in Fig. 9.

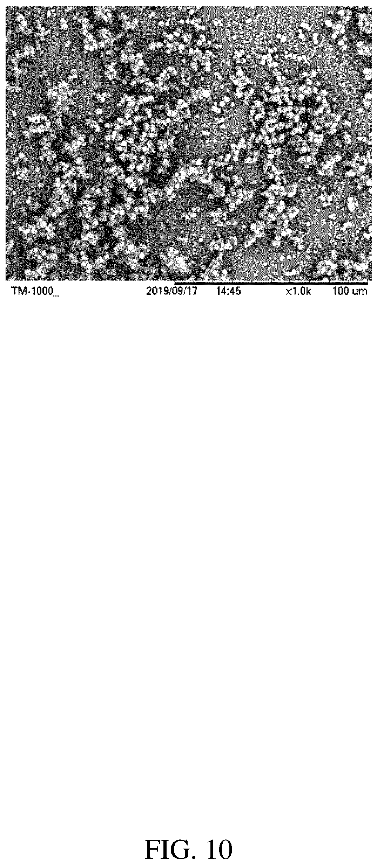

In another embodiment, an inflated 5.0 x 40 mm Passeo 35 balloon was dip coated in a solution of 0.25% polyvinyl alcohol (PVA; EMD PIN 1.41350.1000, Lot K50570650, 85-89% hydrolysis, 3.4-4.6 mPa*s viscosity) prepared by adding the PVA to a tared vial using stainless steel spatula. HPLC grade water was added at the required amount into the vial using a glass measuring pipette. A stir bar was added into the vial and the vial was capped. PVA was dissolved by stirring the solution for 3-4 hours in an 80°C water bath. After cooling of the PVA to ambient temperature the balloon was dip coated as described in Example XIV CRC-015 was formulated and coated onto the PVA coated balloon as described in Example I. The balloon surface was examined by SEM with resulting SEM image showing drug spheres produced shown in Fig. 10.

### XV. In vivo data supporting CRC-015 microsphere coated balloon

The left and right profunda (deep femoral artery) and superficial femoral artery (SFA) of Yucatan mini pigs were treated with CRC-015 DCB (Example XIV) to assess the in-vivo transfer of the drug coating to arterial vessel. It is accepted that this porcine model is a general predictor of human performance. Prior to treatment with CRC-015 DCB, the target vessels were first pre-dilated with uncoated balloon to mimic surgical procedures typically performed in human clinic settings. The carotid artery of the animal was surgically exposed and ligated after administration of anesthesia. An 8F introducer sheath was inserted and secured. The 18000 guide wire and 8F guiding catheter was inserted through the introducer sheath and advanced to the target vessels. Angiographic images of the vessels were obtained with contrast media and the vessel size was measured using calibration markers.

Under fluoroscopic guidance, an uncoated sterile 5.0 x 40 mm pre dilatation balloon catheter was inserted over the guide wire through the guiding catheter, advanced to the target site, and inflated for 30 seconds. The pre-dilatation balloon was deflated and withdrawn. The 5.0 x 40 mm drug coated balloon catheter was then advanced to the dilated vessel segment and inflated for 120 seconds. The drug coated balloon was deflated and withdrawn. Angiograms of treated vessels were obtained with contrast media after every balloon inflation.

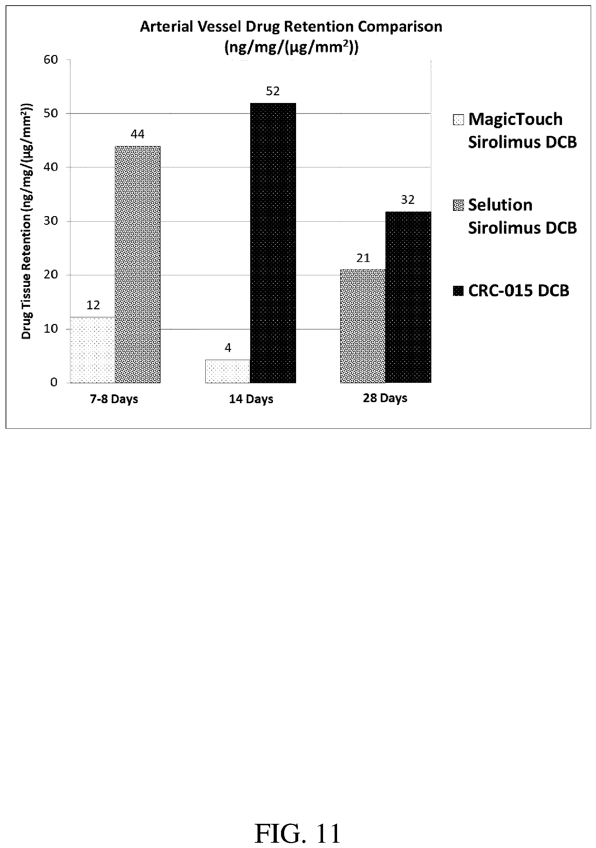

A total of six animals were treated with CRC-015 DCB. Three animals (six femoral arteries per test group) were euthanized at 14 days and three animals (six femoral arteries per test group) at 28 days. Treated vessels were recovered at necropsy and assayed for drug content by HPLC quantitation. The tissue drug retention of CRC-015 at 14 and 28 days was normalized as a function of ng drug/mg tissue/µg/mm2 drug dose on the balloon and reported as ng/mg/(µg/mm2). Arterial vessel drug retention of CRC-015 DCB is compared to results reported for MagicTouch Sirolimus DCB (Concept Medical, Cardiovascular Innovation Pipeline @ EuroPCR, May 26, 2010) and Selution Sirolimus DCB (Zeller, et al. MAC, Dec 07-09, 2017) as shown in Fig. 11.

These results indicate the improved in vivo performance of a product of this invention as compared to the more chemically complex and complicated products referenced.

In further aspects of the present invention, coating of the sphere forming compositions can be accomplished by spraying, rolling, brushing, dip coating, direct fluid or ink-jet application of solution to the substrate. This invention also allows for the production of drug spheres containing two or more drugs together on a medical device to treat a variety of disease conditions.

## Claims

1. A method of manufacturing a microsphere composition comprising at least one drug, comprising the steps of (1) dissolving the at least one drug in at least one solvent to form a hydrophobic mixture, (2) depositing the dissolved hydrophobic mixture onto a substrate and (3) evaporating the deposited, dissolved hydrophobic mixture from the substrate to yield the microsphere composition, wherein the method further comprises a step (1b) prior to step (2) of applying a water soluble material, to the substrate and drying the water soluble material, wherein water soluble material is a protein which is selected from water soluble human serum proteins.

2. The method of claim 1, wherein the at least one solvent is dry, and in particular contains less than 7% water.

3. The method of claim 1 or 2, wherein the method further comprises a step (1a) of cleaning the substrate with an organic solvent.

4. The method of one of the preceding claims, wherein the water soluble material is human serum albumin.

5. The method of one of the preceding claims, wherein the method further comprises a cleansing step by plasma treatment prior to step (1b) or (2).

6. A medical device having a coating layer comprising or consisting of a plurality of drug microspheres, wherein the medical device comprises a primer layer comprising a water soluble material and wherein water soluble material is a protein which is selected from water soluble human serum proteins.

7. The medical device of claim 6, wherein the medical device is a balloon catheter having a balloon.

8. The medical device of claim 6 or 7, wherein the microspheres have an average diameter of 1 to 20 µm.

9. The medical device of one of claims 6 to 8, wherein the coating layer consists of a plurality of drug microspheres.

10. The medical device of one of claims 6 to 9, wherein the drug microsphere comprise or consist of at least one of rapamycin (sirolimus), everolimus, zotarolimus, biolimus, novolimus, myolimus, temsirolimus and/or rapamycin derivatives, including any pharmaceutically acceptable salts and hydrates, as well as any stereoisomers, mixtures of stereoisomers and racemates as well as a macrocyclic triene immunosuppressive compound, including any pharmaceutically acceptable salts and hydrates, as well as any stereoisomers, mixtures of stereoisomers and racemates, has the following structure: 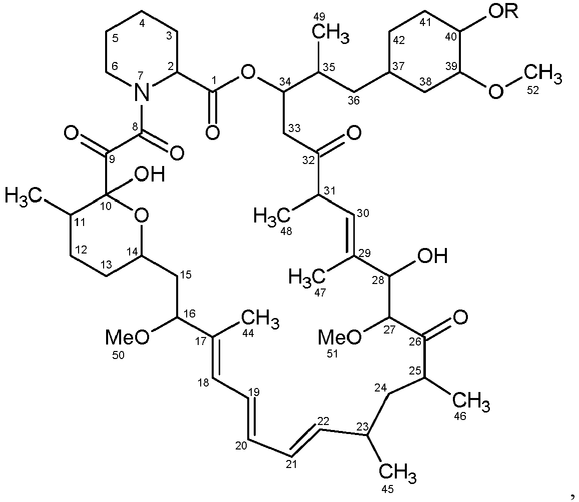 where R is C(O)-(CH₂)ₙ-X, n is 0, 1 or 2, X is a cyclic hydrocarbon having 3-8 carbons and optionally contains one or more unsaturated bonds.

11. The medical device of one of claims 6 to 10, the water soluble material is a protein having a molecular weight of between 50 to 200 kD.

12. The medical device of one of claims 11, wherein the water soluble material is human serum albumin.

13. The medical device of one of claims 7 to 12, wherein the balloon of the balloon catheter is made of a polyamide material.

14. The medical device of one of claims 11 to 13, wherein the medical device is a balloon catheter having a balloon and wherein the primer layer is applied on the balloon and the coating layer consisting of drug microspheres is applied on the primer layer and wherein the balloon does not comprise any additional layer.

## Patentansprüche

1. Verfahren zum Herstellen einer Mikrokugelzusammensetzung, die mindestens ein Medikament umfasst, die folgenden Schritte umfassend: (1) Auflösen des mindestens einen Medikaments in mindestens einem Lösungsmittel, um eine hydrophobe Mischung zu bilden, (2) Abscheiden der aufgelösten hydrophoben Mischung auf einem Substrat und (3) Verdampfen der abgeschiedenen, aufgelösten hydrophoben Mischung aus dem Substrat, um die Mikrokugelzusammensetzung zu erhalten, wobei das Verfahren ferner vor Schritt (2) einen Schritt (1b) umfasst, in dem ein wasserlösliches Material auf das Substrat aufgebracht wird und das wasserlösliche Material getrocknet wird, wobei das wasserlösliche Material ein Protein ist, das ausgewählt ist aus wasserlöslichen humanen Serumproteinen.

2. Verfahren nach Anspruch 1, wobei das mindestens eine Lösungsmittel trocken ist und insbesondere weniger als 7 % Wasser enthält.

3. Verfahren nach Anspruch 1 oder 2, wobei das Verfahren ferner einen Schritt (1a) umfasst, in dem das Substrat mit einem organischen Lösungsmittel gereinigt wird.

4. Verfahren nach einem der vorangehenden Ansprüche, wobei das wasserlösliche Material humanes Serumalbumin ist.

5. Verfahren nach einem der vorangehenden Ansprüche, wobei das Verfahren ferner einen Reinigungsschritt durch eine Plasmabehandlung vor Schritt (1b) oder (2) umfasst.

6. Medizinische Vorrichtung mit einer Überzugsschicht, die eine Vielzahl von Medikamenten-Mikrokugeln umfasst oder von solchen gebildet wird, wobei die medizinische Vorrichtung eine Primerschicht umfasst, die ein wasserlösliches Material umfasst, und wobei das wasserlösliche Material ein Protein ist, das aus wasserlöslichen humanen Serumproteinen ausgewählt ist.

7. Medizinische Vorrichtung nach Anspruch 6, wobei die medizinische Vorrichtung ein Ballonkatheter mit einem Ballon ist.

8. Medizinische Vorrichtung nach Anspruch 6 oder 7, wobei die Mikrokugeln einen durchschnittlichen Durchmesser von 1 bis 20 µm aufweisen.

9. Medizinische Vorrichtung nach einem der Ansprüche 6 bis 8, wobei die Überzugsschicht von einer Vielzahl von Medikamenten-Mikrokugeln gebildet wird.

10. Medizinische Vorrichtung nach einem der Ansprüche 6 bis 9, wobei die Medikamenten-Mikrokugel mindestens eines von Rapamycin (Sirolimus), Everolimus, Zotarolimus, Biolimus, Novolimus, Myolimus, Temsirolimus und/oder Rapamycinderivate, einschließlich jeglicher pharmazeutisch unbedenklicher Salze und Hydrate sowie jeglicher Stereoisomere, Mischungen aus Stereoisomeren und Razematen, sowie eine makrozyklische, immunsuppressive Trienverbindung, einschließlich jeglicher pharmazeutisch unbedenklicher Salze und Hydrate, sowie jeglicher Stereoisomere, Mischungen aus Stereoisomeren und Razematen, umfasst oder von solchen gebildet wird, mit der folgenden Struktur: 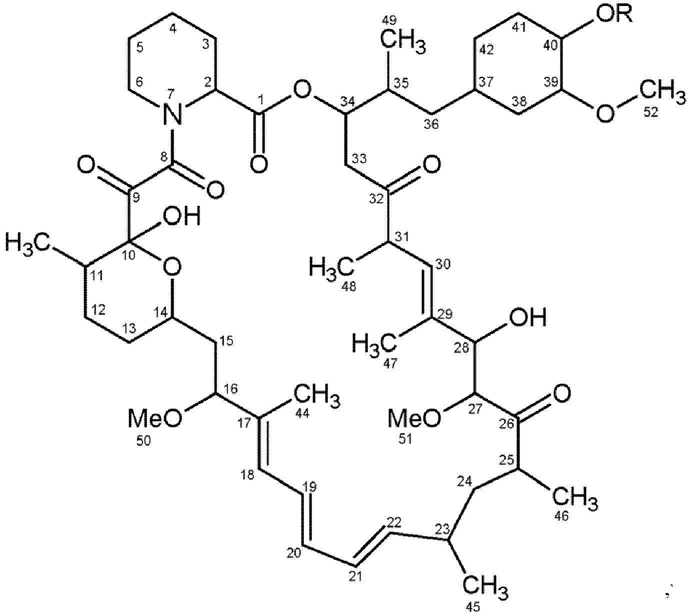
wo R für C(O)-(CH₂)ₙ-X steht, n
für 0, 1 oder 2 steht, X ein zyklischer Kohlenwasserstoff mit 3-8 Kohlenstoffatomen ist und optional eine oder mehrere ungesättigte Bindungen enthält.

11. Medizinische Vorrichtung nach einem der Ansprüche 6 bis 10, wobei das wasserlösliche Material ein Protein mit einem Molekulargewicht zwischen 50 und 200 kD ist.

12. Medizinische Vorrichtung nach einem der Ansprüche 11, wobei das wasserlösliche Material humanes Serumalbumin ist.

13. Medizinische Vorrichtung nach einem der Ansprüche 7 bis 12, wobei der Ballon des Ballonkatheters aus Polyamidmaterial gefertigt ist.

14. Medizinische Vorrichtung nach einem der Ansprüche 11 bis 13, wobei die medizinische Vorrichtung ein Ballonkatheter mit einem Ballon ist, und wobei die Primerschicht auf den Ballon aufgebracht ist und die Überzugsschicht von Medikamenten-Mikrokugeln gebildet wird, die auf die Grundierschicht aufgebracht sind, und wobei der Ballon keine zusätzliche Schicht umfasst.
| Fig. 11 | |
|---|---|
| Arterial Vessel... | Medikamentenretentionsvergleich für arterielle Gefäße |
| Days | Tage |
| Drug Tissue Retention | Medikamentenretention im Gewebe |

## Revendications

1. Procédé de fabrication d'une composition de microsphères comprenant au moins un médicament, comprenant les étapes consistant à (1) dissoudre l'au moins un médicament dans au moins un solvant pour former un mélange hydrophobe, (2) déposer le mélange hydrophobe dissous sur un substrat et (3) évaporer le mélange hydrophobe déposé et dissous à partir du substrat pour obtenir la composition de microsphères, dans lequel le procédé comprend en outre une étape (1b) avant l'étape (2) d'application d'une matière hydrosoluble au substrat et de séchage de la matière hydrosoluble, dans lequel la matière hydrosoluble est une protéine qui est choisie parmi les protéines sériques humaines hydrosolubles.

2. Procédé selon la revendication 1, dans lequel l'au moins un solvant est anhydre, et en particulier contient moins de 7 % d'eau.

3. Procédé selon la revendication 1 ou 2, dans lequel le procédé comprend en outre une étape (1a) de nettoyage du substrat avec un solvant organique.

4. Procédé selon l'une des revendications précédentes, dans lequel la matière hydrosoluble est l'albumine sérique humaine.

5. Procédé selon l'une des revendications précédentes, dans lequel le procédé comprend en outre une étape de purification par traitement au plasma avant l'étape (1b) ou (2).

6. Dispositif médical ayant une couche de revêtement comprenant ou constituée d'une pluralité de microsphères de médicament, dans lequel le dispositif médical comprend une sous-couche comprenant une matière hydrosoluble et dans lequel la matière hydrosoluble est une protéine qui est choisie parmi les protéines sériques humaines hydrosolubles.

7. Dispositif médical selon la revendication 6, dans lequel le dispositif médical est un cathéter à ballonnet comportant un ballonnet.

8. Dispositif médical selon la revendication 6 ou 7, dans lequel les microsphères présentent un diamètre moyen de 1 à 20 µm.

9. Dispositif médical selon l'une des revendications 6 à 8, dans lequel la couche de revêtement est constituée d'une pluralité de microsphères de médicament.

10. Dispositif médical selon l'une des revendications 6 à 9, dans lequel les microsphères de médicament comprennent ou sont constituées d'au moins un parmi la rapamycine (sirolimus), l'évérolimus, le zotarolimus, le biolimus, le novolimus, le myolimus, le temsirolimus et/ou les dérivés de rapamycine, y compris les éventuels sels et hydrates pharmaceutiquement acceptables, ainsi que les éventuels stéréoisomères, mélanges de stéréoisomères et racémates ainsi qu'un composé immunosuppresseur de type triène macrocyclique, y compris les éventuels sels et hydrates pharmaceutiquement acceptables, ainsi que les éventuels stéréoisomères, mélanges de stéréoisomères et racémates, présentant la structure suivante : 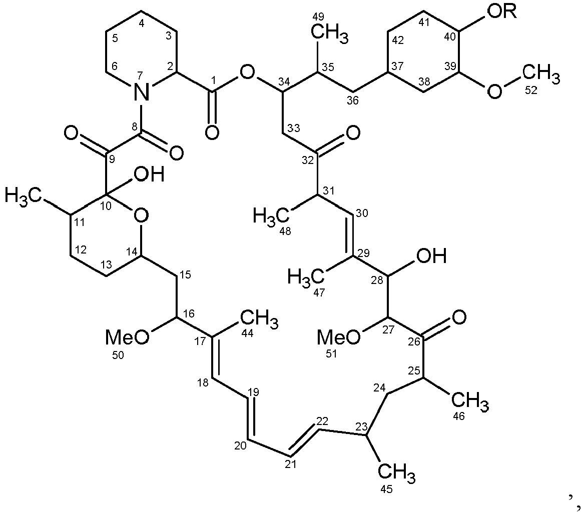 où R représente C(O)-(CH₂)ₙ-X, n représente 0, 1 ou 2, X représente un hydrocarbure cyclique ayant 3 à 8 carbones et contient éventuellement une ou plusieurs liaisons insaturées.

11. Dispositif médical selon l'une des revendications 6 à 10, la matière hydrosoluble étant une protéine ayant une masse moléculaire comprise entre 50 et 200 kD.

12. Dispositif médical selon l'une des revendications 11, dans lequel la matière hydrosoluble est l'albumine sérique humaine.

13. Dispositif médical selon l'une des revendications 7 à 12, dans lequel le ballonnet du cathéter à ballonnet est fabriqué en un matériau de type polyamide.

14. Dispositif médical selon l'une des revendications 11 à 13, dans lequel le dispositif médical est un cathéter à ballonnet comportant un ballonnet et dans lequel la sous-couche est appliquée sur le ballonnet et la couche de revêtement constituée de microsphères de médicament est appliquée sur la sous-couche et dans lequel le ballonnet ne comprend pas de couche supplémentaire.
